(19) Europäisches Patentamt
European Patent Office
Office européen des brevets

(11) Veröffentlichungsnummer: **0 062 789**
**B1**

# (12) EUROPÄISCHE PATENTSCHRIFT

(45) Veröffentlichungstag der Patentschrift: **03.07.85**

(51) Int. Cl.⁴: **C 07 C 7/11**, B 01 D 53/14, C 07 C 9/04

(21) Anmeldenummer: **82102378.5**

(22) Anmeldetag: **23.03.82**

## (54) Verfahren zur Abtrennung von kondensierbaren aliphatischen Kohlenwasserstoffen und sauren Gasen aus Erdgasen.

(30) Priorität: **31.03.81 DE 3112661**

(43) Veröffentlichungstag der Anmeldung: **20.10.82 Patentblatt 82/42**

(45) Bekanntmachung des Hinweises auf die Patenterteilung: **03.07.85 Patentblatt 85/27**

(84) Benannte Vertragsstaaten: **AT BE DE FR GB IT NL SE**

(56) Entgegenhaltungen:
**DE - A - 2 928 858**
**US - A - 2 596 692**
**US - A - 3 824 766**
**US - A - 3 837 143**

(73) Patentinhaber: **BASF Aktiengesellschaft, Carl-Bosch-Strasse 38, D-6700 Ludwigshafen (DE)**

(72) Erfinder: **Wagner, Eckhart, Dr., Jakobsgarten, D-6700 Ludwigshafen (DE)**
Erfinder: **Wagner, Ulrich, Dr., Knospstrasse 7, D-6703 Limburgerhof (DE)**
Erfinder: **Volkamer, Klaus, Dr., Heidelberger Ring 21, D-6710 Frankenthal (DE)**
Erfinder: **Vodrazka, Wolfgang, Dr., Uhlandstrasse 8, D-6713 Freinsheim (DE)**

**EP 0 062 789 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Abtrennung von kondensierbaren aliphatischen Kohlenwasserstoffen und sauren Gasen, wie $H_2S$, $CO_2$ und/oder COS, aus diese kondensierbaren aliphatischen Kohlenwasserstoffe und sauren Gase enthaltenden Erdgasen durch Behandlung der Erdgase mit Dialkylethern von Polyethylenglykolen.

Es ist bekannt, beispielsweise aus Oil and Gas Journal, Jan. 21, 1980, Seiten 66 bis 70, oder der US-Patentschrift 3824766, saure Komponenten aus Erdgasen unter Verwendung von Dialkylethern von Polyethylenglykolen, welche als physikalische Lösungsmittel wirken, zu entfernen. Diese Arbeitsweise ist hervorragend für die Entfernung von sauren Komponenten, besonders für die selektive Entfernung von schwefelhaltigen Komponenten, aus sogenannten trockenen Erdgasen geeignet, d.h. Erdgasen, deren Kohlenwasserstoffanteil im wesentlichen aus Methan besteht. Dagegen ist das bekannte Verfahren nicht geeignet für die Entfernung von sauren Komponenten aus sogenannten «nassen» Erdgasen, d.h. Erdgasen, die neben Methan auch höhere aliphatische Kohlenwasserstoffe enthalten.

Vielmehr wurden für die Aufarbeitung von nassen Erdgasen in den technischen Erdgas-Waschanlagen bisher chemische Lösungsmittel, z.B. wässrige Lösungen von Alkanolaminen wie Diethanolamin, verwendet. Bei der Behandlung mit den wässrigen Alkanolamin-Lösungen wurden aus dem nassen Erdgas die sauren Komponenten entfernt, während die höheren aliphatischen Kohlenwasserstoffe die Erdgas-Wäsche durchliefen und anschliessend durch Kondensation aus dem Erdgas gewonnen wurden. Vor der Kondensationsstufe musste jedoch noch eine aufwendige Trocknungsstufe zwischengeschaltet werden, um den bei der Behandlung mit der wässrigen Alkanolamin-Lösung vom Erdgas aufgenommenen Wasserdampf wieder zu entfernen. Ein weiterer Nachteil der chemischen Lösungsmittel liegt darin, dass diese in der Regel bezüglich der Abtennung der schwefelhaltigen sauren Komponenten nur eine geringe Selektivität gegenüber $CO_2$ aufweisen.

Wegen der den chemischen Lösungsmitteln anhaftenden Nachteile bei der Aufarbeitung von Erdgasen bestand ein erheblicher Bedarf nach einem Verfahren, bei dem die hervorragenden Lösungsmitteleigenschaften der als physikalische Lösungsmittel wirkenden Dialkylether von Polyethylenglykolen auch bei der Aufarbeitung von nassen Erdgasen genutzt werden können.

Es wurde nun ein vorteilhaftes Verfahren gefunden zur Abtennung von kondensierbaren aliphatischen Kohlenwasserstoffen und sauren Gasen wie $H_2S$, $CO_2$ und COS aus diese kondensierbaren aliphatischen Kohlenwasserstoffe und sauren Gase enthaltenden Erdgasen, welches dadurch gekennzeichnet ist, dass man

a) das Erdgas zunächst in einer ersten Absorptionsstufe mit Dialkylethern von Polyethylenglykolen als Lösungsmittel behandelt und dabei eine Absorption der kondensierbaren aliphatischen Kohlenwasserstoffe bewirkt,

b) danach das aus der ersten Absorptionsstufe abgezogene Erdgas in einer zweiten Absorptionsstufe unter erhöhtem Druck mit Dialkylethern von Polyethylenglykolen als Lösungsmittel behandelt, wobei die sauren Gase ganz oder teilweise absorbiert werden,

c) das aus der ersten Absorptionsstufe erhaltene, mit den kondensierbaren aliphatischen Kohlenwasserstoffen beladene Lösungsmittel in einer Extraktionsstufe mit Wasser behandelt unter Ausbildung einer die kondensierbaren aliphatischen Kohlenwasserstoffe enthaltenden Kohlenwasserstoffphase und einer Wasser-/Dialkylether-Phase und die Kohlenwasserstoffphase von der Wasser-/Dialkylether-Phase abtrennt,

d) das aus der zweiten Absorptionsstufe erhaltene, mit sauren Gasen beladene Lösungsmittel in einer Regenerierstufe durch Entspannung und/oder Ausstreifen regeneriert, und

e) das regenerierte Lösungsmittel zur Absorption zurückführt.

Nach dem neuen Verfahren können in einfacher Weise neben der Entfernung der im nassen Erdgas enthaltenen Schwefelverbindungen auch die darin enthaltenen höheren aliphatischen Kohlenwasserstoffe ganz oder teilweise entfernt werden, so dass das nach der Aufarbeitung erhaltene Erdgas in die Gasverteilungsnetze eingespeist werden kann, ohne dass es bei tiefen Temperaturen zur störenden Verflüssigung und Abscheidung der höheren aliphatischen Kohlenwasserstoffe kommt. Die abgetrennten höheren aliphatischen Kohlenwasserstoffe sind wichtige Ausgangsstoffe für petrochemische Verfahren, z.B. für die Herstellung von Ethylen.

Die erfindungsgemäss einzusetzenden Erdgase enthalten im allgemeinen neben der Hauptkomponente Methan und den sauren Gasen wie $H_2S$, $CO_2$ und/oder COS unterschiedliche Mengen an Kohlenwasserstoffen aus der homologen Reihe der aliphatischen Kohlenwasserstoffe mit 2 bis 15, vorzugsweise 2 bis 10 Kohlenstoffatomen. In der Regel beträgt die Konzentration dieser aliphatischen Kohlenwasserstoffe im Erdgas 0,1 bis 10 Mol.-%.

Die Menge des aus dem Gas zu entfernenden Schwefelwasserstoffs kann ebenfalls innerhalb weiter Grenzen variieren. Im allgemeinen weisen die einzusetzenden Gase einen Schwefelwasserstoffgehalt von mindestens 5 Vol.-ppm, vorzugsweise mindestens 10 Vol.-ppm, insbesondere mindestens 100 Vol.-ppm und in der Regel bis zu 50 Vol.-%, vorzugsweise bis zu 40 Vol.-%, insbesondere bis zu 30 Vol.-% auf. Als weitere schwefelhaltige saure Komponente können die einzusetzenden Erdgase COS enthalten, z.B. in Mengen von 3 Vol.-ppm bis 2 Vol.-%.

Neben dem Schwefelwasserstoff enthalten die einzusetzenden Gase häufig als weiteres Sauergas Kohlendioxid, z.B. in Mengen von 0,01 bis 60 Vol.-%, vorzugsweise 0,1 bis 45 Vol.-%, insbesondere 0,1 bis 30 Vol.-%.

Als Dialkylether von Polyethylenglykolen kommen im allgemeinen solche der allgemeinen Formel

$$R^1 - O[- CH_2 - CH_2 - O]_n - R^2$$

in Betracht, in der $R^1$ und $R^2$ jeweils einen verzweigten oder geradkettigen $C^1$- bis $C^5$-Alkylrest, vorzugsweise $C_1$- bis $C_4$-Alkylrest, und n zur Kennzeichnung der Anzahl der Ethylenglykolgruppen eine ganze Zahl von 2 bis 9, vorzugsweise 3 bis 8, bedeuten, wobei $R^1$ und $R^2$ gleich oder verschieden sein können. Zweckmässig werden Dialkylether verwendet, in denen $R^1$ und $R^2$ Kohlenwasserstoffreste bedeuten. Geeignete Reste $R^1$ und $R^2$ sind z.B. der Methyl-, Ethyl-, n-Propyl-, Isopropyl-, n-Butyl-, Isobutyl-, tert.-Butyl-Rest, die Amyl-Reste wie tert.-Amyl-Rest.

Geeignete Dialkylether von Polyethylenglykolen sind z.B. der Dimethyl-, Methyl-ethyl-, Methyl-n-propyl-, Methyl-iso-propyl-, Methyl-n-butyl-, Methyl-isobutyl-, Methyl-tert.-butyl-, Methyl-tert.-amyl-, Diethyl-, Ethyl-n-propyl-, Ethyl-isopropyl-, Ethyl-n-butyl-, Ethyl-isobutyl-, Ethyl-tert.-butyl-, Ethyl-tert.-amyl-, Di-n-propyl-, Diisopropyl-, n-Propyl-isopropyl-, n-Propyl-n-butyl-, n-Propyl-isobutyl-, n-Propyl-tert.-butyl-, n-Propyl-tert.-amyl-, Isopropyl-n-butyl-, Isopropyl-isobutyl-, Isopropyl-tert.-butyl-, Isopropyl-tert.-amyl-ether. Vorzugsweise werden die Dimethyl- und Methylisopropylether verwendet. Man kann Dialkylether von Polyethylenglykolen mit der gleichen Anzahl n der Ethylenglykolgruppen verwenden. In der Praxis werden jedoch in der Regel Mischungen von Dialkylethern von Polyethylenglykolen mit im allgemeinen 2 bis 8 Ethylenglykolgruppen verwendet.

Erfindungsgemäss wird das zu behandelnde Erdgas zur Absorption der kondensierbaren aliphatischen Kohlenwasserstoffe zunächst in einer ersten Absorptionsstufe mit Dialkylethern von Polyethylenglykolen als Lösungsmittel behandelt. Zweckmässig weist das Lösungsmittel in der ersten Absorptionsstufe einen Wassergehalt von 0,5 bis 8 Gew.-%, vorzugsweise 0,5 bis 5 Gew.-% auf. Die erste Absorptionsstufe kann unter Atmosphärendruck betrieben werden. Zweckmässig wird in der ersten Absorptionsstufe jedoch erhöhter Druck, im allgemeinen ein Druck zwischen 5 und 150 bar, vorzugsweise zwischen 10 und 130 bar, insbesondere zwischen 20 und 120 bar aufrechterhalten. In der Regel betragen die Temperaturen in der ersten Absorptionszone −20 bis +60°C, vorzugsweise −20 bis +40°C. Die erste Absorptionsstufe wird vorteilhaft als Absorptionskolonne, im allgemeinen als Füllkörper- oder Bodenkolonne, betrieben, wobei das Lösungsmittel der Absorptionskolonne zweckmässig in der oberen Hälfte, vorzugsweise im oberen Drittel, zugeführt und im allgemeinen im Gegenstrom zu dem zu behandelnden Gas geführt wird.

Das aus der ersten Absorptionsstufe abgezogene Erdgas wird anschliessend zur Absorption der sauren Komponenten wie $H_2S$, $CO_2$ und/oder COS in einer zweiten Absorptionsstufe unter erhöhtem Druck ebenfalls mit Dialkylethern von Polyethylenglykolen als Lösungsmittel behandelt. Zweckmässig weist das der zweiten Absorptionsstufe zugeführte Lösungsmittel einen Wassergehalt auf, der höher ist als der Wassergehalt in dem der ersten Absorptionsstufe zugeführten Lösungsmittel. Im allgemeinen beträgt der Wassergehalt in dem der zweiten Absorptionsstufe zugeführten Lösungsmittel 1,5 bis 14 Gew.-%, vorzugsweise 2 bis 10 Gew.-%. Zweckmässig wird in der zweiten Absorptionsstufe der gleiche Dialkylether von Polyethylenglykolen bzw. das gleiche Dialkylethergemisch verwendet wie in der ersten Absorptionsstufe.

Die zweite Absorptionsstufe wird unter erhöhtem Druck betrieben. Im allgemeinen wird in der zweiten Absorptionsstufe ein Druck zwischen 5 und 150 bar, vorzugsweise zwischen 10 und 130 bar, insbesondere zwischen 20 und 120 bar aufrechterhalten. Vorteilhaft werden erste und zweite Absorptionsstufe bei dem gleichen Druck betrieben.

In der Regel betragen die Temperaturen in der zweiten Absorptionsstufe −20 bis +60°C, vorzugsweise −20 bis +40°C. Die zweite Absorptionsstufe wird vorteilhaft als Absorptionskolonne, im allgemeinen als Füllkörper- oder Bodenkolonne, betrieben, wobei das Lösungsmittel der Absorptionskolonne zweckmässig in der oberen Hälfte, vorzugsweise im oberen Drittel, zugeführt und im allgemeinen im Gegenstrom zu dem zu behandelnden Gas geführt wird.

Falls das in der zweiten Absorptionsstufe zu behandelnde Erdgas als saure Gase Schwefelwasserstoff und Kohlendioxid enthält, können beide sauren Gase gemeinsam in der zweiten Absorptionsstufe entfernt werden. Eine vorteilhafte Ausführungsform des erfindungsgemässen Verfahrens besteht jedoch darin, dass Schwefelwasserstoff, falls das Gas Kohlendioxid enthält, selektiv in der zweiten Absorptionszone entfernt wird. Die Lösungsmittelmenge, die in der zweiten Absorptionsstufe angewendet wird, beträgt im allgemeinen ein Mehrfaches der Lösungsmittelmenge, die in der ersten Absorptionsstufe benötigt wird, z.B. die 2- bis 100fache, vorzugsweise 5- bis 50fache Lösungsmittelmenge.

Das aus der ersten Absorptionsstufe erhaltene, mit den kondensierbaren aliphatischen Kohlenwasserstoffen beladene Lösungsmittel wird in einer Extraktionsstufe mit Wasser behandelt. Zweckmässig wird das mit den Kohlenwasserstoffen beladene Lösungsmittel mit Wasser vermischt, wobei sich eine die kondensierbaren aliphatischen Kohlenwasserstoffe enthaltende Kohlenwasserstoffphase und eine Wasser-/Dialkylether-Phase ausbilden. Durch Abtrennen der Kohlenwasserstoffphase können die aliphatischen Kohlenwasserstoffe in flüssiger und reiner Form erhalten werden. Im allgemeinen wird das mit den Kohlenwasserstoffen beladene Lösungsmittel mit einer solchen Menge Wasser behandelt, dass der Wassergehalt der sich bildenden Wasser-/Dialkylether-Phase zwischen 16 und 90 Gew.-%, vorzugsweise zwischen 20 und 80 Gew.-%, liegt. Die Extraktionsstufe wird zweckmässig in der Weise betrieben, dass das mit den Kohlenwasserstoffen beladene Lösungsmittel und das in die Extraktionsstufe eingeleitete Wasser im Gegenstrom geführt werden. Als Extraktionsapparate können beispielsweise solche vom Typ des Mixer-Settlers oder Gegenstromkolonnen mit rotierenden Einbauten (z.B. Rotating Disc Contactor) oder auch pulsierte Füllkörperkolonnen eingesetzt werden. Die Extraktionsstufe kann bei Atmosphärendruck oder bei erhöhtem Druck, z.B. bei Drücken von 1 bis 30 bar, betrieben werden.

Das aus der zweiten Absorptionsstufe erhaltene, mit sauren Gasen beladene Lösungsmittel wird in einer Regenerierstufe unter Austreiben der sauren Gase regeneriert. Die Regenerierung kann durch Entspannung des mit sauren Gasen beladenen Lösungsmittels in einer Entspannungszone auf einen Druck unterhalb des Absorptionsdrucks oder durch Ausstreifen der sauren Gase in einer Desorptionszone, zweckmässig unter Verwendung von Wasserdampf als Ausstreifmittel, erfolgen. In einer bevorzugten Ausführungsform wird das mit den sauren Gasen beladene Lösungsmittel zunächst in einer Entspannungszone unter Entspannungsverdampfung (Flashverdampfung) entspannt. Das bei der Entspannungsverdampfung anfallende Gasgemisch wird zweckmässig in die zweite Absorptionszone zurückgeführt, wodurch Verluste von teilweise gelösten Wertprodukten wie Methan, Ethan gering gehalten werden. Das aus der Entspannungszone erhaltene Lösungsmittel wird anschliessend in einer nachgeschalteten Desorptionszone mit Wasserdampf ausgestreift, wobei die gelösten sauren Gase praktisch vollständig aus dem Lösungsmittel ausgestreift werden. Wenn die zweite Absorptionsstufe unter selektiver $H_2S$-Abtrennung betrieben wird, kann beim Ausstreifen mittels Wasserdampf in der Desorptionsstufe ein hochkonzentriertes $H_2S$-Gas erzeugt werden, das beispielsweise in einer Claus-Anlage zu elementarem Schwefel aufgearbeitet werden kann.

In einer speziellen Ausführungsform des Verfahrens wird die unter Ausstreifen mit Wasserdampf betriebene Desorptionszone und die Extraktionsstufe durch einen geschlossenen Wasser-/Lösungsmittelkreislauf verknüpft. Dies geschieht zweckmässig in der Weise, dass man für die Behandlung des mit den kondensierbaren aliphatischen Kohlenwasserstoffen beladenen Lösungsmittels in der Extraktionsstufe das Wasser verwendet, das als kondensiertes Wasser beim Ausstreifen des mit den sauren Gasen beladenen Lösungsmittels mit Wasserdampf in der Desorptionszone zweckmässig durch Abkühlen der ausgestreiften, grössere Mengen Wasserdampf enthaltenden sauren Gase in einer der Desorptionszone nachgeschalteten Kondensationszone erhalten wird. Gleichzeitig wird, um den Kreislauf zu schliessen, das nach Abtrennen der Wasser-/Dialkylether-Phase in der Extraktionsstufe erhaltene Wasser-/Dialkylether-Gemisch in die Desorptionszone zurückgeführt, in der das Gemisch zweckmässig als flüssiger Rücklauf am Kopf der Desorptionszone zugegeben wird.

Das am Sumpf der Desorptionszone abgezogene regenerierte Lösungsmittel wird zur Absorption zurückgeführt. Dabei wird es der zweiten Absorptionsstufe im allgemeinen mit dem gleichen Wassergehalt zugeführt, mit dem es aus der Desorptionszone abgezogen worden ist. Dagegen wird im allgemeinen der Wassergehalt des regenerierten Lösungsmittels, das auf die erste Absorptionsstufe gegeben wird, vor der Zugabe zur ersten Absorptionsstufe, zweckmässig durch Abdestillieren des Wassers, abgesenkt.

Nachfolgend werden weitere Einzelheiten der Erfindung anhand eines Ausführungsbeispiels, dessen Verfahrensablauf in der Figur schematisch dargestellt ist, erläutert.

Ein Schwefelwasserstoff, Kohlendioxid und aliphatische Kohlenwasserstoffe enthaltendes Gas der in der Tabelle angegebenen Zusammensetzung wird über Leitung 1 unter Druck in den Sumpf der Absorptionskolonne 2 gegeben. Gleichzeitig wird über Leitung 29 eine im Kühler 28 abgekühlte Lösungsmittelmenge, bestehend aus einer Mischung von Dialkylethern von Polyethylenglykolen und einer Restmenge Wasser, auf den Kopf der Absorptionskolonne gegeben. Dies Lösungsmittel, das im Gegenstrom zum Gas geführt wird, löst den überwiegenden Anteil der normalerweise flüssigen Kohlenwasserstoffe und geringe Mengen der sauren Komponenten $H_2S$ und $CO_2$ aus dem zu behandelnden Gasgemisch heraus. Das Gas wird über Leitung 3 am Kopf der Kolonne 2 abgezogen und anschliessend in den Sumpf der Absorptionskolonne 4 eingeführt. Die Kolonne 4 wird ebenfalls mit einem gekühlten Lösungsmittelstrom über Leitung 24 beaufschlagt. Im allgemeinen wird die Menge des Stromes über Leitung 24 ein vielfaches der Menge des Stromes über Leitung 29 betragen. Am Kopf der Kolonne 4 wird ein Reingas über Leitung 5 abgezogen, das nur noch einen geringen Gehalt an höheren Kohlenwasserstoffen und den sauren Komponenten $H_2S$ und/oder $CO_2$ aufweist. Der mit den höheren Kohlenwasserstoffen beladene Lösungsmittelstrom 6 wird über Ventil 7 in einen Entspannungsverdampfungsbehälter (Flashbehälter) 8 auf Atmosphärendruck entspannt. Dabei werden die normalerweise gasförmigen Komponenten, die sich in der Absorptionskolonne 2 gelöst haben, desorbiert und über Leitung 9 dem Kompressor 10 zugeführt. Das beladene Lösungsmittel aus Absorptionskolonne 4 wird über Leitung 11 und Ventil 12 in den Entspannungsverdampfungsbehälter 13 entspannt. Dort werden je nach Entspannungsdruck eine mehr oder weniger grosse Menge an gelöstem Gas desorbiert. Das Entspannungsgas wird über Leitung 14 mit dem vorkomprimierten Entspannungsgas aus Behälter 8 vermischt, in Kompressor 15 auf den Absorptionsdruck in Kolonne 4 komprimiert, in Kühler 16 abgekühlt und anschliessend in die Absorptionskolonne 4 zurückgeführt. Das am Boden des Entspannungsbehälters 13 abgezogene Lösungsmittel wird über Leitung 17 zum Wärmetauscher 18 und nach Entspannung im Ventil 19 auf den Kopf der Desorptionskolonne 20 geleitet, in der die sauren Komponenten durch aufsteigenden Wasserdampf ausgestreift und über Leitung 33 nach Kondensation des Wasserdampfes in Kondensator 34 und Abscheidung des kondensierten Wassers in Behälter 35 über Leitung 36 abgegeben werden. Der zum Ausstreifen der sauren Verbindungen benötigte Wasserdampf wird im Aufkocher 21 durch indirekten Kontakt des regenerierten Lösungsmittels mit einem Heizmedium erzeugt und in der Desorptionskolonne 20 aufwärts zum auszustreifenden Lösungsmittel geführt. Die Hauptmenge des vom Boden der Kolonne 20 abgezogenen Lösungsmittels wird über Leitung 22 in den Wärmetauschern 18 und 23 abgekühlt und über Leitung 24 auf den Kopf der Absorptionskolonne 4 gegeben. Ein kleinerer Teil wird über Leitung 25 einer Kolonne 26 zugeführt, in der der Wassergehalt des Lösungsmittels durch Destillation herabgesetzt wird. Das Lösungsmittel mit vermindertem Wassergehalt wird am Boden der Kolonne 26 abgezogen und nach Kühlung in Wärmetauscher 28 über Leitung 29 auf den Kopf des Absorbers 2 gegeben. Das Wasser, das die Kolonne 26 als Kopfprodukt verlässt, wird im Kondensator 30 verflüssigt und teils über Leitung 31 als flüssiger Rücklauf auf den Kopf der Kolonne 26 gegeben und teils mit dem kondensierten Wasser aus Behälter 35 vermischt. Die vereinigten Wasserströme werden über Leitung 38 in den oberen Teil der Extraktionskolonne 39 eingeführt. Das Gemisch aus Polyethylenglykoldialkylethern und Kohlenwasserstoffen, das im unteren Teil der Kolonne 39 eingeführt wird, vermischt sich mit dem Wasser, wodurch eine Phasentrennung zwischen Wasser-Dialkylether-Gemisch einerseits und den flüssigen Kohlenwasserstoffen andererseits stattfindet. Die flüssigen Kohlenwasserstoffe scheiden sich am Kopf der Kolonne ab und werden dort über Leitung 41 abgezogen. Die Kolonne ist über die Entlüftungsleitung 40 mit dem Behälter 35 verbunden. Der am Boden der Kolonne abgezogene wässrige Dialkyletherstrom wird über Leitung 43 auf den Kopf der Desorptionskolonne 20 gegeben. Mit Hilfe der Pulsationspumpe 42 wird für eine gute Vermischung der wässrigen und organischen Phase in der Extraktionskolonne 39 gesorgt.

Tabelle
Typische Zusammensetzung eines höhere Kohlenwasserstoffe enthaltenden sauren Erdgases

| Komponente | Konzentration (Mol-%) |
|---|---|
| $N_2$ | 3.2 |
| $CO_2$ | 4.3 |
| $H_2S$ | 1.0 |
| $CH_4$ | 84.9 |
| $C_2H_6$ | 5.3 |
| $C_3H_8$ | 0.65 |
| $i\text{-}C_4H_{10}$ | 0.32 |
| $n\text{-}C_4H_{10}$ | 0.12 |
| $C_5H_{12}$ | 0.09 |
| $C_6H_{14}$ | 0.05 |
| $C_7H_{16}$ | 0.05 |
| $C_{8+}$ | 0.02 |

**Patentansprüche**

Verfahren zur Abtrennung von kondensierbaren aliphatischen Kohlenwasserstoffen und sauren Gasen, wie $H_2S$, $CO_2$ und COS, aus diese kondensierbaren aliphatischen Kohlenwasserstoffe und sauren Gase enthaltenden Erdgasen, dadurch gekennzeichnet, dass man

a) das Erdgas zunächst in einer ersten Absorptionsstufe mit Dialkylethern von Polyethylenglykolen als Lösungsmittel behandelt und dabei eine Absorption der kondensierbaren aliphatischen Kohlenwasserstoffe bewirkt,

b) danach das aus der ersten Absorptionsstufe abgezogene Erdgas in einer zweiten Absorptionsstufe unter erhöhtem Druck mit Dialkylethern von Polyethylenglykolen als Lösungsmittel behandelt, wobei die sauren Gase ganz oder teilweise absorbiert werden,

c) das aus der ersten Absorptionsstufe erhaltene, mit den kondensierbaren aliphatischen Kohlenwasserstoffen beladene Lösungsmittel in einer Extraktionsstufe mit Wasser behandelt unter Ausbildung einer die kondensierbaren aliphatischen Kohlenwasserstoffe enthaltenden Kohlenwasserstoffphase und einer Wasser-/Dialkylether-Phase und die Kohlenwasserstoffphase von der Wasser-/Dialkylether-Phase abtrennt,

d) das aus der zweiten Absorptionsstufe erhaltene, mit sauren Gasen beladene Lösungsmittel in einer Regenerierstufe durch Entspannung und/oder Ausstreifen regeneriert, und

e) das regenerierte Lösungsmittel zur Absorption zurückführt.

2. Verfahren nach Anspruch 1, dadurch gekennzeichnet, dass das aus der zweiten Absorptionsstufe erhaltene, mit sauren Gasen beladene Lösungsmittel in der Weise in der Regenerierstufe regeneriert wird, dass das beladene Lösungsmittel zunächst in einer Entspannungszone durch Entspannen des beladenen Lösungsmittels auf einen Druck unterhalb des Absorptionsdruckes von einem Teil der gelösten sauren Gase und anschliessend in einer Desorptionszone durch Ausstreifen mit Wasserdampf von einem weiteren Teil der gelösten sauren Gasen befreit wird.

3. Verfahren nach Anspruch 1 und 2, dadurch gekennzeichnet, dass der Wassergehalt des Lösungsmittels in der ersten Absorptionsstufe zwischen 0,5 und 8 Gew.-% liegt.

4. Verfahren nach Anspruch 1 bis 3, dadurch gekennzeichnet, dass der Wassergehalt des Lösungsmittels in der zweiten Absorptionsstufe zwischen 1,5 und 14 Gew.-% liegt.

5. Verfahren nach Anspruch 1 bis 4, dadurch gekennzeichnet, dass der Wassergehalt der Wasser-/Dialkylether-Phase in der Extraktionsstufe zwischen 16 und 90 Gew.-% beträgt.

6. Verfahren nach Anspruch 2, dadurch gekennzeichnet, dass man den Wassergehalt des regenerierten Lösungsmittels, das auf die erste Absorptionsstufe gegeben wird, vor der Zugabe zur ersten Absorptionsstufe durch Abdestillieren von Wasser absenkt.

7. Verfahren nach Anspruch 2, dadurch gekennzeichnet, dass man das aus der ersten Absorptionsstufe erhaltene, mit den kondensierbaren aliphatischen Kohlenwasserstoffen beladene Lösungsmittel zur Abtrennung der Kohlenwasserstoffe als Kohlenwasserstoffphase in der Extraktionsstufe mit dem beim Ausstreifen mit Wasserdampf in der Absorptionszone anfallenden kondensierten Wasser vermischt.

8. Verfahren nach Anspruch 2, dadurch gekennzeichnet, dass man das nach Abtrennen der Wasser-/Dialkylether-Phase in der Extraktionsstufe erhaltene Wasser-/Dialkylether-Gemisch in die Desorptionszone gibt.

9. Verfahren nach Anspruch 2, dadurch gekennzeichnet, dass man zur Abtrennung der Kohlenwasserstoffe aus dem mit den kondensierbaren aliphatischen Kohlenwasserstoffen beladenen Lösungsmittel das mit den Kohlenwasserstoffen beladene Lösungsmittel in einer Gegenstromextraktionskolonne mit Wasser behandelt.

**Claims**

1. A process for removing condensable aliphatic hydrocarbons and acidic gases, such as $H_2S$, $CO_2$ and COS, from natural gas containing these, wherein

a) the natural gas is initially treated with polyethylene glycol dialkyl ethers, as the solvent, in a first absorption stage to effect absorption of the condensable aliphatic hydrocarbons,

b) the natural gas drawn off from the first absorption stage is then treated with polyethylene glycol dialkyl ethers, as the solvent, under superatompsheric pressure in a second absorption stage, the acidic gases being completely or partly absorbed,

c) the solvent charged with condensable aliphatic hydrocarbons which is obtained from the first stage is treated with water in an extraction stage to form a hydrocarbon phase containing the condensable aliphatic hydrocarbons and an aqueous dialkyl ether phase, and the hydrocarbon phase is separated from the aqueous dialkyl ether phase,

d) the solvent charged with acidic gases which is obtained from the second absorption stage is regenerated by expansion and/or stripping in a regeneration stage, and

e) the regenerated solvent is recycled to the absorption.

2. A process as claimed in claim 1, wherein the solvent charged with acidic gases which is obtained from the second absorption stage is regenerated in the regeneration stage in such a manner that the charged solvent is first freed from some of the dissolved acidic gases in an expansion zone by expansion of the charged solvent to a pressure below the absorption pressure, and is then freed from more of the dissolved acidic gases in a desorption zone by stripping with steam.

3. A process as claimed in claims 1 and 2, wherein the solvent in the first absorption stage contains from 0.5 to 8% by weight of water.

4. A process as claimed in claims 1 to 3, wherein the solvent in the second absorption stage contains from 1.5 to 14% by weight of water.

5. A process as claimed in claims 1 to 4, wherein the aqueous dialkyl ether phase in the extraction stage contains from 16 to 90% by weight of water.

6. A process as claimed in claims 2, wherein the water content of the regenerated solvent introduced into the first absorption stage is decreased by distilling off water beforehand.

7. A process as claimed in claim 2, wherein the solvent charged with condensable aliphatic hydrocarbons which is obtained from the first absorption stage and which is used for separating off the hydrocarbons as a hydrocarbon phase in the extraction stage is mixed with the condensed water obtained during stripping with steam in the absorption zone.

8. A process as claimed in claim 2, wherein the mixture of water and dialkyl ether obtained in the extraction stage after separation of the aqueous dialkyl ether phase is introduced into the desorption zone.

9. A process as claimed in claim 2, wherein the solvent charged with hydrocarbons is treated with water in a countercurrent extraction column in order to separate the hydrocarbons from the solvent charged with condensable aliphatic hydrocarbons.

**Revendications**

1. Procédé pour extraire les hydrocarbures aliphatiques condensables et les gaz acides tels que $H_2S$, $CO_2$ et COS, de gaz naturels contenant des hydrocarbures aliphatiques condensables et des gaz acides, caractérisé en ce que:

a) le gaz naturel est soumis dans un premier stade d'absorption à un traitement avec des éthers dialkyliques de polyéthylène-glycols comme solvant, provoquant une absorption des hydrocarbures aliphatiques condensables;

b) le gaz naturel soutiré du premier stade d'absorption est soumis dans un deuxième stade d'absorption, sous pression accrue, à un nouveau traitement avec des éthers dialkyliques de polyéthylène-glycols comme solvant, qui provoque une absorption totale ou partielle des gaz acides;

c) le solvant chargé des hydrocarbures aliphatiques condensables, provenant du premier stade d'absorption, est traité dans un stade d'extraction avec de l'eau, ce qui conduit à la formation d'une phase contenant les hydrocarbures aliphatiques condensables et d'une phase éther dialkylique – eau, la phase des hydrocarbures étant ensuite séparée de la phase éther dialkylique-eau;

d) le solvant chargé des gaz acides, provenant du deuxième stade d'absorption, est régénéré dans un stade de régénération par détente et (ou) entraînement, et

e) le solvant régénéré est recyclé dans le stade d'absorption.

2. Procédé suivant la revendication 1, caractérisé en ce que le solvant chargé des gaz acides, provenant du deuxième stade d'absorption, est régénéré dans le stade de régénération en le soumettant d'abord, dans une zone de détente, à une détente jusqu'à une pression inférieure à la pression d'absorption, qui en élimine une partie des gaz acides dissous, puis dans une zone de désorption à un entraînement à la vapeur d'eau, qui élimine une partie supplémentaire des gaz acides dissous.

3. Procédé suivant la revendication 1 et la revendication 2, caractérisé en ce que, dans le premier stade d'absorption, la teneur en eau du solvant est comprise entre 0,5 et 8% en poids.

4. Procédé suivant les revendications 1 à 3, caractérisé en ce que, dans le deuxième stade d'absorption, la teneur en eau du solvant est comprise entre 1,5 et 14% en poids.

5. Procédé suivant les revendications 1 à 4, caractérisé en ce que, dans le stade d'extraction, la teneur en eau de la phase éther dialkylique-eau est comprise entre 16 et 90% en poids.

6. Procédé suivant la revendication 2, caractérisé en ce que, la teneur en eau du solvant régénéré, à introduire dans le premier stade d'absorption, est abaissée avant l'introduction de celui-ci dans le premier stade d'absorption par l'élimination d'une partie de l'eau par distillation.

7. Procédé suivant la revendication 2, caractérisé en ce que, pour la séparation des hydrocarbures sous forme d'une phase d'hydrocarbures dans le stade d'extraction, on mélange au solvant chargé des hydrocarbures aliphatiques condensables, provenant du premier stade d'absorption, l'eau condensée, provenant de l'entraînement à la vapeur d'eau dans la zone d'absorption.

8. Procédé suivant la revendication 2, caractérisé en ce que, le mélange éther dialkylique-eau, obtenu après la séparation de la phase éther dialkylique-eau dans le stade d'extraction, est amené dans la zone de désorption.

9. Procédé suivant la revendication 2, caractérisé en ce que, pour en extraire les hydrocarbures, on traite le solvant chargé des hydrocarbures aliphatiques condensables, dans une colonne d'xtraction à contre-courant, avec de l'eau.

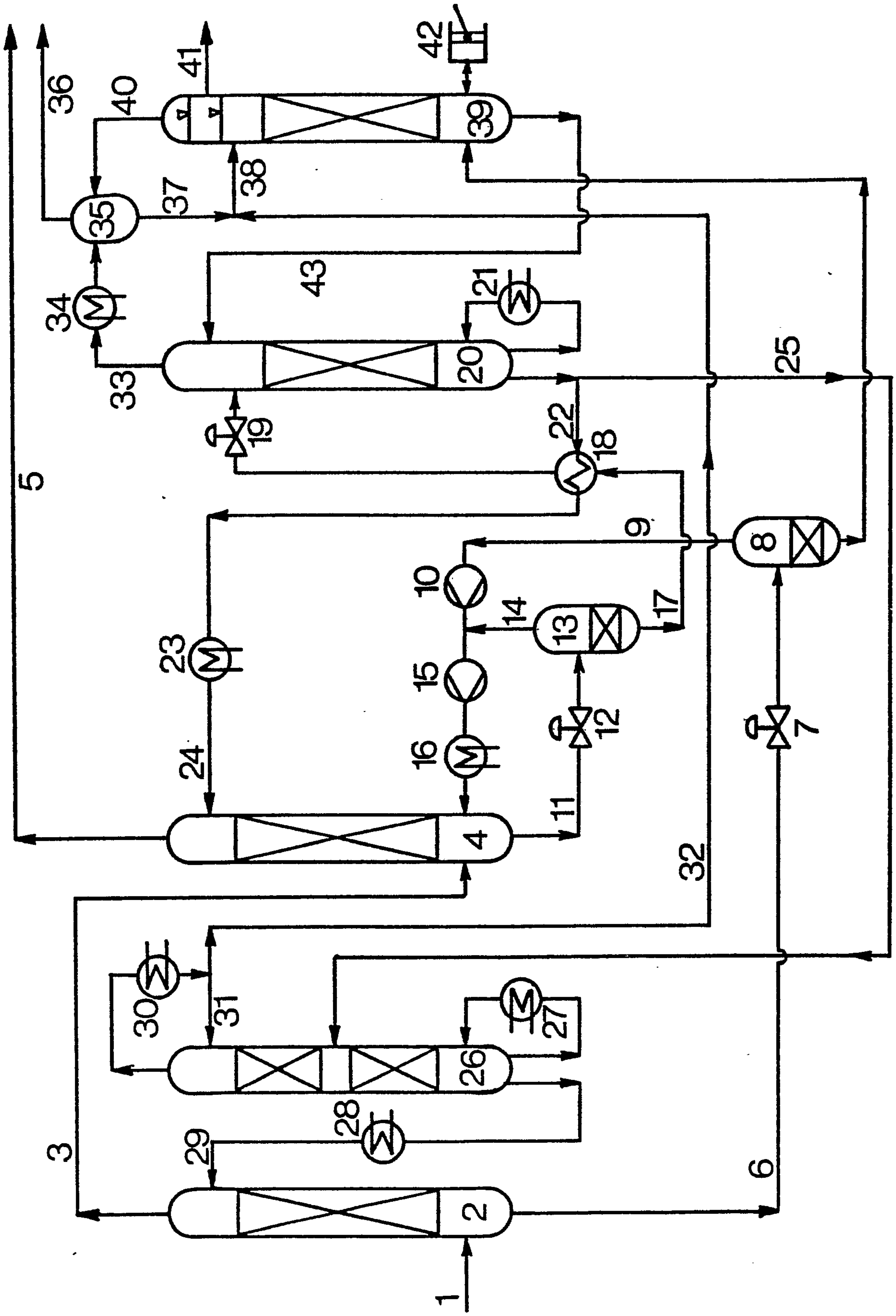
1
2
3
4
5
6
7
8
9
10
11
12
13
14
15
16
17
18
19
20
21
22
23
24
25
26
27
28
29
30
31
32
33
34
35
36
37
38
39
40
41
42
43